Europäisches Patentamt

**European Patent Office**

Office européen des brevets

(11) Numéro de publication: **0 119 893 B1**

(12) **FASCICULE DE BREVET EUROPEEN**

(45) Date de publication du fascicule du brevet:
**18.10.89**

(51) Int. Cl.⁴: **A 61 K 39/40,** G 01 N 33/569

(21) Numéro de dépôt: **84400348.3**

(22) Date de dépôt: **20.02.84**

(54) Anticorps monoclonaux anti-Légionella, procédé d'obtention et application au dosage de Légionella pneumophila.

(30) Priorité: **21.02.83 FR 8302789**

(43) Date de publication de la demande:
**26.09.84 Bulletin 84/39**

(45) Mention de la délivrance du brevet:
**18.10.89 Bulletin 89/42**

(84) Etats contractants désignés:
**AT BE CH DE FR GB IT LI LU NL SE**

(56) Documents cités:
**BIOLOGICAL ABSTRACTS, vol. 76, no. 8, 1983, page 6237, no. 57107, Philadelphia, PA., US; K.K. SETHI et al.: "Hybridoma-derived monoclonal immunoglobulin M antibodies to Legionella pneumophila serogroup 1 with diagnostic potential" & J. CLIN MICROBIOL 17(6): 953-957, 1983**
**BIOLOGICAL ABSTRACTS, vol. 73, 1982, no. 32777, Philadelphia, PA., US; CH.R. RINALDO Jr. et al.: "Growth of the Pittsburgh pneumonia agent in animal cell cultures" & INFECT IMMUN 33(3): 939-943, 1981**

Le dossier contient des informations techniques présentées postérieurement au dépôt de la demande et ne figurant pas dans le présent fascicule.

(73) Titulaire: **CENTRE NATIONAL DE LA RECHERCHE SCIENTIFIQUE (CNRS), 15, Quai Anatole France, F-75007 Paris (FR)**
Titulaire: **INSTITUT PASTEUR Fondation reconnue d'utilité publique, 28 rue du Docteur Roux, F-75724 Paris Cédex 15 (FR)**

(72) Inventeur: **Guillet, Jean Gérard, 2, rue de la Pierre Levée, F-75011 Paris (FR)**
Inventeur: **Hoebeke, Johan, 28, rue Pierrelais, F-92320 Chatillon (FR)**
Inventeur: **Tram, Cuong, 51, Boulevard Auguste Blanqui, F-75013 Paris (FR)**
Inventeur: **Strosberg, Donny A., 66, rue de Javel, F-75015 Paris (FR)**

(74) Mandataire: **Phélip, Bruno et al, c/o Cabinet Harlé & Phélip 21, rue de La Rochefoucauld, F-75009 Paris (FR)**

ACTORUM AG

## Description

La présente invention a pour objet des anticorps monoclonaux anti-Legionella; elle concerne également un procédé d'obtention de ces anticorps, les lignées de cellules hybrides murines produisant de tels anticorps, ainsi que les applications biologiques de ceux-ci, notamment à la prévention et au diagnostic des pneumonies, au dépistage et à l'identification dans le milieu environnant des bactéries Legionella.

L'introduction des tests de diagnostic sérologique pour l'étude de l'étiologie de la pneumonie atypique a montré que l'agent pathogène principal de cette maladie était la bactérie Legionella pneumophila (Macfarlane, J.T. et al 1982. Hospital Study of adult community acquired pneumonia. Lancet 1982; 255–258). Cette maladie a été constatée de façon sporadique ou sous la forme d'épidémie en particulier dans le domaine hospitalier. Il s'est donc avéré intéressant de développer une méthodologie standard pour le diagnostic, qui soit capable non seulement de détecter les anticorps humains anti-Legionella qui apparaissent tardivement lors d'une infection, mais également de détecter des quantités mineures de bactéries elles-mêmes ou d'antigènes bactériens, récoltés dans les expectorations, les lavages et les prélèvements broncho-trachéitiques et les urines.

Ce type de diagnostic n'est fiable que si l'on dispose d'anticorps contre des antigènes spécifiques de Legionella, qui ne provoquent pas de réactions croisées avec d'autres bactéries.

On a maintenant obtenu des lignées de cellules hybrides qui produisent des anticorps monoclonaux anti-Legionella, qui peuvent être utilisés pour le diagnostic des pneumonies. Ces anticorps monoclonaux ont une affinité élevée, stable et constante vis-à-vis des antigènes de Legionella, ils peuvent être utilisés pour le dosage quantitatif direct de l'agent infectieux dans les premières phases de la maladie et pour le dosage des anticorps anti-Legionella dans le sang des malades au cours des phases ultérieures de la maladie.

La présente invention a donc pour objet des lignées de cellules hybrides murines anti-Legionella pneumophila de sérogroupe I, déposées à la Collection Nationale de Cultures de Micro-organismes de l'Institut Pasteur, Paris (France), sous les n° I-219 et I-220, le 14 février 1983, produisant des anticorps monoclonaux anti-Legionella ayant un titre élevé et une spécificité rigoureuse.

Les lignées de cellules hybrides murines selon l'invention sont obtenues par fusion de cellules de myélome de souris avec des splénocytes de souris immunisées contre les Legionella, dont l'espèce Legionella pneumophila.

La fusion de cellules de myélome de souris avec des lymphocytes de souris immunisées a été proposée pour la première fois en 1975 par KOHLER et MILSTEIN [Nature 256 495–497 (1975)]. La technique de fusion, également dénommée «technique des hybridomes» a été depuis largement utilisée pour la production d'anticorps monoclonaux. Cependant, jusqu'à présent, on n'a jamais, à la connaissance de la Demanderesse, fabriqué des anticorps anti-Legionella.

Sous son aspect général, le procédé selon l'invention pour l'obtention de lignées de cellules hybrides murines anti-Legionella comprend les étapes suivantes:

1) d'immunisation de souris avec une quantité donnée de bactéries Legionella tuées;

2) de prélèvement de la rate des souris immunisées et séparation des splénocytes;

3) de fusion des splénocytes ainsi obtenus avec des cellules de myélome de souris en présence d'un promoteur de fusion;

4) de culture des cellules hybrides obtenues dans un milieu sélectif sur lequel ne se développent pas les cellules de myélome non fusionnées, et en présence d'éléments nutritifs appropriés;

5) de sélection des cellules produisant l'anticorps désiré et clonage de ces cellules.

Le procédé selon l'invention s'applique aux bactéries de la famille des Legionellaceae, dont on connaît actuellement un seul genre, le genre Legionella qui comporte plusieurs espèces, notamment Legionella pneumophila.

La première étape du procédé de l'invention consiste en une immunisation de souris avec des bactéries Legionella tuées par un moyen quelconque, par exemple par un agent chimique, tel que le formol, ou par la chaleur. On utilise des bactéries d'un sérogroupe donné, par exemple Legionella pneumophila sérogroupe I. Pour plus de détails sur de telles bactéries, on pourra se référer à la publication ci-après:

McDade, J.F. et al 1977, Legionnaire's disease: isolation of a bacterium and demonstration of its role in other respiratory diseases – N. Engl. J. Med. 297 1197–1203, cité dans la présente description à titre de référence.

A titre d'exemples de bactéries qui conviennent dans le procédé de l'invention, on peut citer notamment: Legionella pneumoniae de sérogroupe I, souche Legionella pneumophila, Philadelphia souche 1 déposée au «Centre for Disease Control» Atlanta, Georgia USA sous le n° ATCC 331.52 le 15 août 1979.

Le procédé selon l'invention va être maintenant décrit en référence à Legionella pneumophila sans pour autant limiter la portée de l'invention à cette seule espèce de Legionella.

L'immunisation des souris est réalisée de manière à stimuler la mémoire des cellules pour la synthèse d'anticorps en évitant la formation d'IgM qui se sont montrés plus labiles que les IgG. Le protocole d'immunisation utilisé selon l'invention est celui qui provoque une réponse secondaire.

Le protocole d'immunisation préféré aux fins de l'invention consiste à injecter une fois par semaine, pendant trois semaines consécutives, par voie intraveineuse, une quantité donnée de bactéries tuées à la chaleur et, trois jours avant le prélèvement et la fusion après une période de repos de trois mois, à faire un rappel par voie intraveineuse avec la même quantité de bactéries tuées.

La quantité de bactéries utilisée peut être de $10^6$ à $10^9$. On utilise avantageusement 200 millions de bactéries tuées à la chaleur.

Les splénocytes des souris sont récupérés après prélèvement de la rate. A cet effet, on injecte dans la rate du milieu RPMI exempt de sérum et on lave les cellules trois fois dans du RPMI exempt de sérum avant la fusion.

Les cellules de myélome de souris utilisées dans le procédé de l'invention sont des cellules de myélome non sécrétantes NS-1. On a utilisé par exemple la lignée de cellules de myélome NS-1 fournie par le Docteur Goodfellow (Imperial Cancer Research Foundation, Londres). Ces cellules ont été sélectionnées pour leur sensibilité à l'aminoptérine et cultivées sur un milieu approprié contenant des substances nutritives. Un tel milieu peut être constitué par exemple d'un milieu RPMI 1640 [un tel milieu est défini par exemple par MOORE et al. Culture of Normal Human leucocytes J.A.M.A. 199 519–524, 1967] contenant 10% de sérum de veau fœtal, 2 mM de glutamine, 1 mM de pyruvate de sodium, 100 UI/ml de pénicilline et 100 µg/ml de streptomycine; ce milieu sera dénommé ci-après milieu RPMI complet. Les cellules de myélome utilisées dans le procédé de l'invention sont des cellules non sécrétantes. Bien que des cellules sécrétantes puissent également être utilisées, il est préférable, pour obtenir des anticorps homogènes, d'utiliser des cellules non sécrétantes.

La fusion des cellules, qui constitue la troisième étape du procédé de l'invention, consiste à mélanger des splénocytes de souris immunisées contre Legionella pneumophila d'un sérogroupe donné et des cellules de myélome de souris non sécrétantes NS-1, selon la technique de KOHLER et MILSTEIN [Nature 256 495–497 (1975)], en présence d'un promoteur de fusion, tel que par exemple un polyéthylène glycol. On utilise avantageusement une cellule de myélome NS-1 pour 10 splénocytes et un polyéthylèneglycol ayant un poids moléculaire de 1000 à raison de 41% en poids par rapport au milieu.

Après incubation à 37°C dans une atmosphère à 10% de $CO_2$, les cellules sont lavées dans du milieu RPMI et remises en suspension dans du milieu RPMI complet, puis on procède à la culture des cellules hybrides résultantes sur un milieu sélectif convenant uniquement pour la croissance des cellules hybrides. Un tel milieu sélectif peut être constitué du milieu complet RPMI auquel on a ajouté 0,1 mM d'hypoxanthine, 0,4 µM d'aminoptérine et 16 µM de thymidine. Il est connu que les cellules de myélome qui sont dépourvues de l'enzyme hypoxanthine-guanine-phosphoribosyl-transférase, ne se reproduisent pas sur les milieux contenant de l'hypoxanthine, de l'aminoptérine et de la thymidine. En conséquence, les cellules de myélome n'ayant pas fusionné ne pourront pas se reproduire sur le milieu sélectif utilisé.

On sélectionne ensuite les surnageants des cultures (en général 15 jours après la fusion) pour déterminer la présence d'anticorps anti-Legionella pneumophila par un test immuno-enzymatique et on sous-clone en présence de cellules nutritives (par exemple de splénocytes). Le sous-clonage est avantageusement effectué par la méthode des dilutions limites selon Oi, V.T. et L.A. Herzenberg 1980. Immunoglobulin-producing hybrid cell lines in «Selected Methods in Cellular Immunology» (Eds. Mishell, R.B. and Shrigi, S.M.) p. 351, Fillman, San Francisco.

Le sous-clonage ci-dessus est avantageusement effectué dans des microplaques; les puits desdites microplaques contenant des clones uniques sont ensuite sélectionnés et leurs surnageants sont testés par exemple par la méthode ELISA pour ne retenir que les clones produisant les anticorps anti-Legionella recherchés, spécifiques de la bactérie utilisée pour l'immunisation des souris, qui ont une haute affinité et qui sont stables.

Pour obtenir des quantités importantes d'anticorps monoclonaux anti-Legionella pneumophila, on injecte les cellules hybrides obtenues (hybridomes) produisant lesdits anticorps à des souris qui ont été préparées («primées») par voie péritonéale avec du tétraméthyl-pentadécane. Après au moins quinze jours, on récolte les ascites qui contiennent des quantités importantes de l'anticorps anti-Legionella pneumophila recherché.

Les anticorps ainsi obtenus sont conservés par congélation à −20°C. Après de fréquentes décongélations et congélations on n'a pas observé de diminution de leur titre.

Les anticorps monoclonaux anti-Legionella obtenus selon l'invention sont des réactifs de choix pour les dépistages des Legionella et le diagnostic des légionelloses. Ils peuvent être utilisés soit pour déceler la présence de faibles quantités de bactéries Legionella pneumophila dans les premières phases de la maladie, soit pour doser les anticorps anti-Legionella dans le sang des malades dans les phases avancées de la maladie, soit pour détecter les antigènes bactériens dans les urines.

Les anticorps monoclonaux selon l'invention ont également des applications dans le domaine thérapeutique. Ils peuvent être mis en œuvre pour la préparation de vaccins. Par exemple, on peut isoler et purifier par chromatographie d'affinité les antigènes reconnus à l'aide d'un anticorps monoclonal selon l'invention. Les antigènes ainsi obtenus peuvent être utilisés pour la fabrication de vaccins selon les méthodes classiques bien connues de l'homme de l'art. On peut alors réaliser une prévention de la maladie par une vaccination systématique des personnes travaillant dans les zones définies comme étant à haut risque (milieu hospitalier, hôtels, etc.).

Les anticorps monoclonaux selon l'invention peuvent également être utilisés comme agent thérapeutique en cas de bactérémie. A cet effet, les anticorps purifiés par chromatographie d'affinité sont stérilisés par filtration (acrodisc 0,2 µm), puis on prépare des solutions injectables contenant les fragments monovalents Fab des anticorps obtenus selon la méthode de PORTER R.R. Biochemical Journal 1959, 73, p. 119–126. Ces solutions peuvent être administrées aux malades par voie intraveineuse sous forme de perfusion.

Les anticorps monoclonaux anti-Legionella selon l'invention sont particulièrement utiles pour effectuer une recherche systématique des bactéries dans le milieu ambiant (par exemple dans l'eau et l'air d'un hôpital) autant du point de vue quantitatif que du point de vue qualitatif. Ils permettent ainsi en particulier d'isoler des nouvelles souches sauvages et d'en effectuer le typage précis. Ils peuvent notamment être utilisés pour détecter la présence de bactéries Legionella retenues sur des filtres fixés sur les arrivées d'eau et d'air dans un milieu ambiant suspect.

Les anticorps monoclonaux selon l'invention peuvent également être couplés à un immunoadsorbant et l'ensemble peut être utilisé comme filtre pour l'isolement et la concentration des bactéries. Ainsi, l'invention concerne également les immuno-adsorbants sur lesquels sont fixés des anticorps monoclonaux selon l'invention; ces immuno-adsorbants conviennent pour le dépistage épidémiologique. Les immuno-adsorbants mis en œuvre sont des immuno-adsorbants classiques, tels que des billes de Sephadex®, sur lesquelles sont immobilisés lesdits anticorps monoclonaux. Les immuno-adsorbants peuvent avantageusement se présenter sous la forme de pastilles immuno-adsorbantes.

Les anticorps selon l'invention conviennent particulièrement bien pour les détections ci-dessus, notamment par les techniques d'immunofluorescence (directe ou indirecte) ou immuno-enzymatique (par exemple méthode ELISA). Ils peuvent également être utilisés dans d'autres techniques de dosage, telles que les techniques radio-immunologiques. L'invention concerne donc également les coffrets de diagnostic immunologique, notamment par voie immuno-enzymatique ou par immunofluorescence, qui contiennent à titre de réactif immunologique principal, un anticorps monoclonal selon l'invention.

De tels coffrets comprennent principalement les réactifs couramment utilisés dans ces types de diagnostic qui sont:

1) pour les tests par immunofluorescence: les anticorps monoclonaux couplés à un fluorochrome, les tampons et les supports appropriés;

2) pour les tests par voie enzymatique: l'anticorps monoclonal, l'enzyme, les agents révélateurs de l'activité enzymatique, les tampons et supports appropriés.

L'invention concerne donc également l'application desdits anticorps au diagnostic de pneumonies et les coffrets de diagnostic par les méthodes immuno-enzymatiques ou d'immunofluorescence, qui contiennent à titre de réactif immunologique un anticorps selon l'invention.

Les anticorps monoclonaux selon l'invention sont spécifiques de la bactérie utilisée pour l'immunisation.

Les anticorps monoclonaux préférés selon l'invention sont les anticorps monoclonaux anti-Legionella pneumophila de sérogroupe I obtenus à partir des lignées de cellules hybrides murines anti-Legionella pneumophila sérogroupe I (Legionella pneumophila Philadelphia Souche 1) qui proviennent de la fusion selon le procédé ci-dessus de splénocytes de souris immunisées contre Legionella pneumophila sérogroupe I et de cellules de myélome de souris non sécrétantes NS-1.

On a réalisé selon l'invention deux fusions distinctes et on a obtenu 28 lignées de cellules hybrides murines, parmi lesquelles on a sélectionné trois lignées II-6, III-1 et III-7 qui ont été sous-clonées. On a alors sélectionné les trois sous-clones suivants II-6-18; III-1-12 et III-7-1. Les lignées de cellules hybrides murines résultant des sous-clonages II-6-18 et III-1-12 ont été déposées dans la Collection Nationale de Culture de Micro-organismes de l'Institut Pasteur à Paris (France) le 14 février 1983 sous les numéros:

I-220 pour la lignée II-6-18,

I-219 pour la lignée III-1-12.

Les anticorps produits par ces lignées de cellules présentent les caractéristiques ci-après:

Les anticorps monoclonaux produits par la lignée cellulaire II-6-18 sont isotypes γ3 et leur point isoélectrique est de 8,7; les anticorps monoclonaux produits par la lignée cellulaire III-1-12 sont isotypes γ-2b et leur point isoélectrique est de 6,5. Les anticorps monoclonaux produits par la lignée cellule III-7-1 sont isotypes γ1.

EXEMPLE 1

Production d'anticorps monoclonaux *anti-Legionella pneumophila* de sérogroupe I.

Dans cet exemple, on a utilisé pour l'immunisation des souris des bactéries Legionella pneumophila de sérogroupe I (Legionella pneumophila Philadelphia souche 1) car 80% ou plus des légionelloses sont provoquées par les bactéries de sérogroupe I (cas constatés en France).

Les bactéries Legionella pneumophila sérogroupe I (souche de référence Philadelphia 1) ont été cultivées dans un milieu constitué essentiellement d'agar complété d'extraits globulaires, d'extraits de levure, de sang de cheval défibrinisé, de cystéine et d'ions ferriques. Le milieu a été tamponné à pH 6,9 par addition d'hydroxyde de potassium et d'autres précurseurs de stimulation de croissance.

Après 2 ou 3 jours d'incubation à 37 °C dans des conditions anaérobies, on a récolté Legionella pneumophila dans un tube contenant une solution physiologique tamponnée au phosphate de pH 7,4. La suspension obtenue a été homogénéisée par vortex et les bactéries ont été tuées par ébullition pendant 20 minutes. La suspension de bactéries tuées a été testée pendant 7 jours pour déceler la présence éventuelle de bactéries vivantes. La suspension de bactéries tuées a été ajustée à 2 × $10^9$ bactéries/ml par mesure de la turbidité à l'aide d'un tube Mac Farland n° 4.

1 – Immunisation

On a immunisé des souris Balb/c selon le protocole défini précédemment, c'est-à-dire que l'on a injecté à des souris Balb/c, une fois par semaine

pendant trois semaines consécutives, 200 millions de bactéries tuées à la chaleur par voie intraveineuse. Après une période de repos de trois mois, les souris ont eu un rappel par voie intraveineuse avec la même quantité de bactéries (200 millions) trois jours avant l'utilisation des splénocytes pour la fusion.

2 – Fusion

On a utilisé des cellules de myélome de souris NS-1 fournies par le Docteur Goodfellow (Imperial Cancer Research Foundation, Londres). Elles ont été sélectionnées pour leur sensibilité à l'aminoptérine et cultivées sur le milieu RPMI contenant 10% de sérum de veau fœtal, 2 mM de glutamine, 1 mM de pyruvate de sodium, 100 UI/ml de pénicilline et 100 µg/ml de streptomycine.

Les splénocytes ont été dispersés par injection du milieu RPMI exempt de sérum dans la rate des souris hyperimmunisées et lavés trois fois dans du milieu RPMI avant fusion.

Les cellules de myélome NS-1 et les splénocytes de deux rates ont été ensuite fusionnés dans un rapport de une cellule de myélome pour 10 splénocytes, en présence de 41% de polyéthylèneglycol d'un poids moléculaire de 1000 (Merck) selon la méthode de KOHLER et MILSTEIN [Nature 256 495–497 (1975)], puis lavés dans le milieu RPMI et remis en suspension dans 100 ml du milieu RPMI complet. Les cellules ont ensuite été redistribuées dans des microplaques Nunclon (24 puits par plaque) à raison de 1 ml/puits contenant 0,22 million de cellules. Après 24 heures, on a ajouté 1 ml/puits de milieu sélectif constitué du milieu RPMI complet contenant 0,1 mM d'hypoxanthine, 0,4 µM d'aminoptérine et 16 µM de Thymidine. Des parties aliquotes de milieu sélectif ont été remplacées les 3ème, 6ème et 10ème jours après la fusion. Après 15 jours, les hybridomes restants ont été cultivés sur le milieu RPMI complet, complété avec de l'hypoxanthine et de la thymidine et 28 jours après la fusion on a recherché pour la première fois la présence d'anticorps anti-Legionella dans les surnageants des cultures. Les clones ont été ensuite cultivés progressivement sur le milieu RPMI complet normal.

Six cultures positives ont été sous-clonées par la méthode des dilutions limites selon Oi, V.T. et L.A. Herzenberg 1980 Immunoglobulin-producing hybrid cell lines in «Selected Methods in Cellular Immunology» (Eds. Mishell, R.B. and Shrigi, S.M. p. 351, Fillman, San Francisco). Les cellules ont été redistribuées dans les puits d'une microplaque Nunclon, à raison de 0,05 ml/puits avec 0,3 million de splénocytes à titre de cellules nutritives. Après 10 jours, les puits contenant des clones uniques ont été sélectionnés et 5 jours plus tard, les surnageants ont été testés pour déterminer la présence d'anticorps anti-*Legionella pneumophila*.

Trois sous-clones issus de trois clones différents, ont été sélectionnés et injectés à des souris Balb/c pour obtenir de grandes quantités d'anticorps. A cet effet, des souris femelles âgées Balb/c ont été «primées» par voie intrapéritonéale avec 0,3 ml de tétraméthyl-pentadécane. Après 4 jours, 20 millions de cellules hybrides ont été injectées aux souris. Après au moins 15 jours, les ascites ont été récoltés et leur activité anti-Legionella a été testée. Selon le mode opératoire ci-dessus, on a effectué deux fusions différentes et sélectionné les sous-clones II-6-18 et III-1-12 qui ont été déposés dans la Collection Nationale de Culture de Micro-organismes sous les numéros I-219 et I-220 comme indiqué précédemment.

EXEMPLE 2
Caractérisation des anticorps obtenus par la méthode ELISA

Les anticorps obtenus selon l'exemple 1 ont été testés pour déterminer leur activité anti-Legionella par la méthode immuno-enzymatique bien connue sous le nom de méthode ELISA.

Les micropuits d'une plaque NUNC-ELISA ont été traités avec 0,05 ml d'une solution de polylysine (vendue par la Société Sigma) 10 µg/ml. Après une heure, à température ambiante les puits ont été vidés et 0,05 ml d'une suspension de bactéries tuées à la chaleur ou formolisées ont été distribués dans chaque puits et évaporés à 50 °C. Pour éviter une adsorption non spécifique des anticorps, les puits ont été en outre saturés à la température ambiante pendant une heure avec de la sérum albumine bovine à 3% dans une solution saline tamponnée au phosphate (PBS). Les puits ont ensuite été lavés une fois avec du PBS contenant 0,1% de sérum albumine bovine.

Les surnageants des cultures d'hybridomes ou les fluides ascitiques à tester ont été dilués dans du PBS contenant 0,1% de sérum albumine bovine; 0,05 ml de ces dilutions a été incubé dans les puits pendant une heure à 37 °C. Après trois lavages dans du PBS contenant 0,1% de sérum albumine bovine (BSA), on a ajouté dans chaque puits 0,05 ml d'immunoglobulines mouton anti-immunoglobulines de souris couplées avec de la peroxydase de raifort (fournie par Institut Pasteur Production, Paris) à la dilution du 1/500. Deux séries de témoins ont été préparées en incubant le conjugué ci-dessus dans des puits:

1) qui n'ont pas été incubés préalablement avec des anticorps anti-Legionella de souris, ou

2) qui ont été incubés avec des immunoglobulines de souris ou des fluides ascitiques non spécifiques vis-à-vis de Legionella.

Après une heure d'incubation avec le conjugué, les puits sont lavés trois fois avec du PBS et on ajoute ensuite le substrat enzymatique [2,5 mM $H_2O_2$ et 2 mM de 2,2′-azinodi-[3-éthyl-benzo-thia-zolin-sulfonate] (ABTS) dans un tampon acétate phosphate pH = 6,5]. Après 30 minutes la densité optique de chaque puits a été lue dans un lecteur optique de la Société ARTEK et les plaques ont été photographiées.

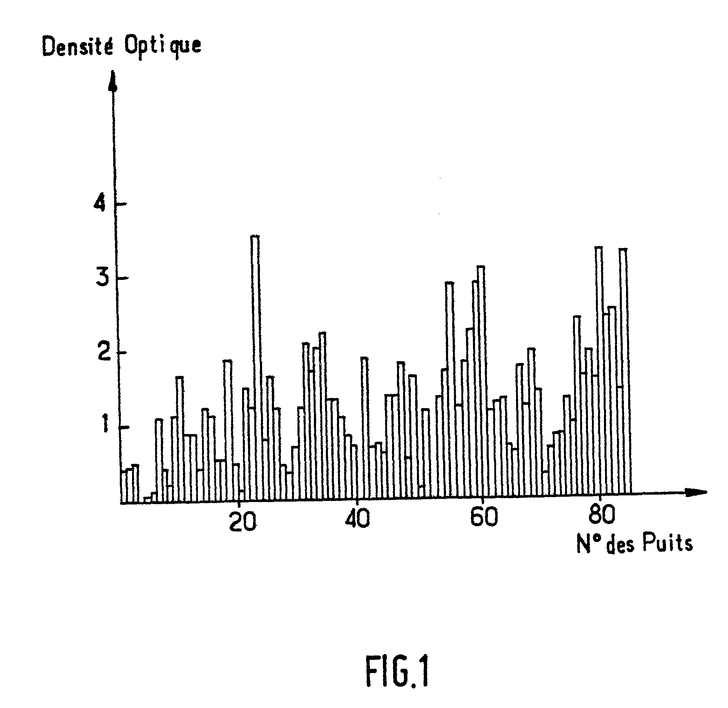

Par cette méthode, on a détecté 28 puits positifs parmi les 85 puits contenant des cellules hybrides en utilisant le critère rigoureux de trois fois par rapport aux témoins pour déterminer le caractère positif. Les résultats obtenus sont portés sur la figure 1, sur laquelle on a porté en ordonnée la densité optique (D.O.) à 405 nm, et en abscisse les puits.

Parmi les 28 puits positifs, 10 ont été testés pour vérifier la sérospécificité des anticorps monoclonaux de l'invention vis-à-vis du sérogroupe I.

A cet effet, on a réalisé le même test que ci-dessus avec des puits incubés avec une suspension de bactéries de sérotypes différents et on a constaté que les surnageants des cultures d'hybridomes étaient nettement positifs vis-à-vis du sérogroupe I, réagissaient au 1/3 ou à la moitié avec un mélange de sérogroupe I, II et III, mais ne réagissaient pas avec un mélange de sérogroupe IV, V et VI ou avec des souches atypiques N, T ou W.

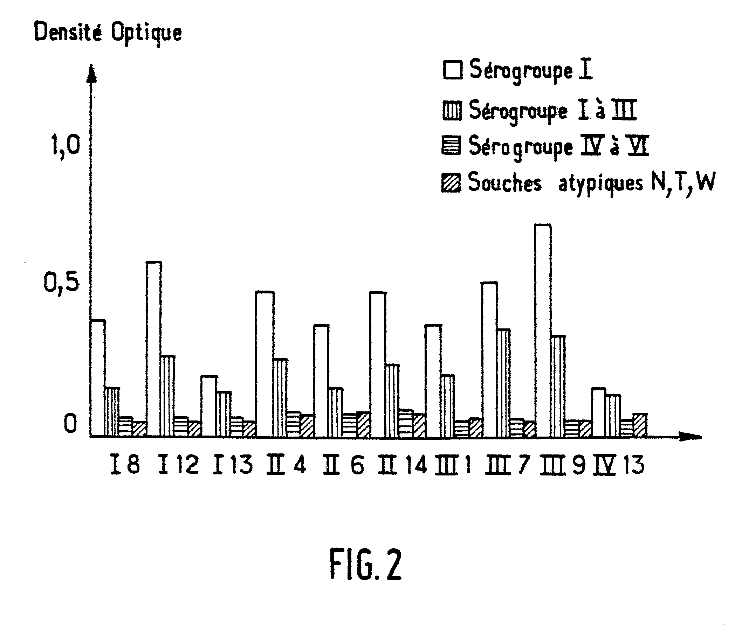

Les résultats obtenus sont portés sur la figure 2 sur laquelle on a porté en ordonnée la densité optique à 405 nm et en abscisse la nature des clones testés.

EXEMPLE 3
Caractérisation sérologique de deux anticorps monoclonaux obtenus à partir de fluides ascitiques

Le sous-clonage de six cultures de cellules hybrides a été effectué pour obtenir des anticorps homogènes. On a sélectionné parmi ces cultures celles qui donnaient des hybrides stables après sous clonage. Deux de ces sous-clones (II-6-18 et III-1-12) ont été injectés à des souris selon le mode opératoire décrit précédemment et on a utilisé les fluides ascitiques obtenus.

Les isotypes de deux anticorps monoclonaux ainsi obtenus ont été testés par la méthode ELISA indirecte en utilisant des anticorps polyclonaux monospécifiques de lapin contre les différentes isotypes d'immunoglobulines murines.

Les fluides ascitiques obtenus après injection du sous-clone II-6-18 ont donné des anticorps anti-Legionella isotype $\gamma$-3 et avec le sous-clone III-1-12 on a obtenu des anticorps isotypes $\gamma$-2b.

Les deux anticorps ainsi obtenus ont été ensuite testés par la méthode ELISA et l'immunofluorescence indirecte contre neuf souches différentes de Legionella. Seule la souche de sérogroupe I a été reconnue par ces deux tests.

On a également utilisé la méthode ELISA pour vérifier l'existence éventuelle de réactions croisées avec d'autres bactéries, en utilisant Klebsiella pneumoniae et Mycoplasma pneumoniae comme antigènes dans le test ELISA. Aucune réaction croisée n'a été obtenue avec les deux anticorps ci-dessus.

EXEMPLE 4
Recherche du titre des fluides ascitiques obtenus avec le clone II-6-18 à des fins de diagnostic

On a recherché la quantité minimale de bactéries donnant un résultat positif par la méthode ELISA.

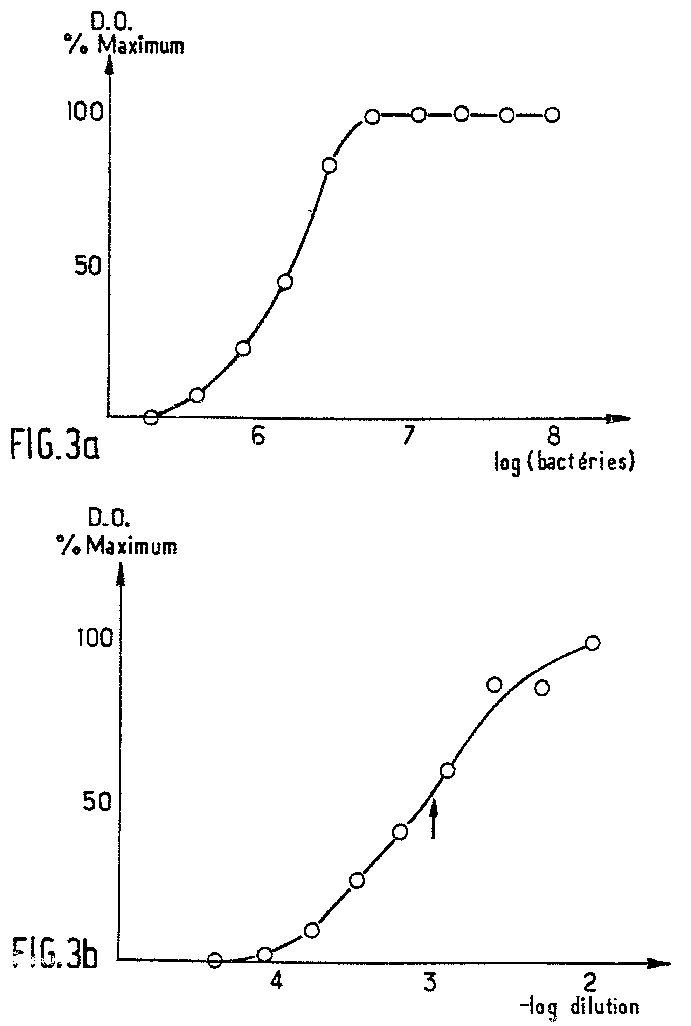

Les résultats obtenus sont portés sur les figures 3a et 3b.

Sur la figure 3a on a porté en ordonnée la densité optique en % par rapport à la valeur maximale obtenue lors de l'essai précédent (% maximum) et en abscisse le logarithme de la quantité de bactéries.

On voit sur cette figure que les quantités de bactéries donnant 50% de la réponse maximale étaient de 2 millions. On voit que des faibles quantités de 0,5 million peuvent également être détectées avec une précision significative.

Le titre du fluide ascitique pour 2 millions de bactéries est illustré sur la figure 3b, sur laquelle on a porté en abscisse le logarithme de la dilution du fluide ascitique et en ordonnée la densité optique (% maximum). On voit que 50% de la réponse maximale sont obtenus avec une dilution de 1/1000.

On peut utiliser les anticorps monoclonaux de deux manières:

— soit dans un test par compétition avec des bactéries Legionella elles-mêmes, pour la quantification de celles-ci;

— soit dans un test par compétition avec des antisérums contenant des anticorps anti-Legionella pour déterminer leurs titres.

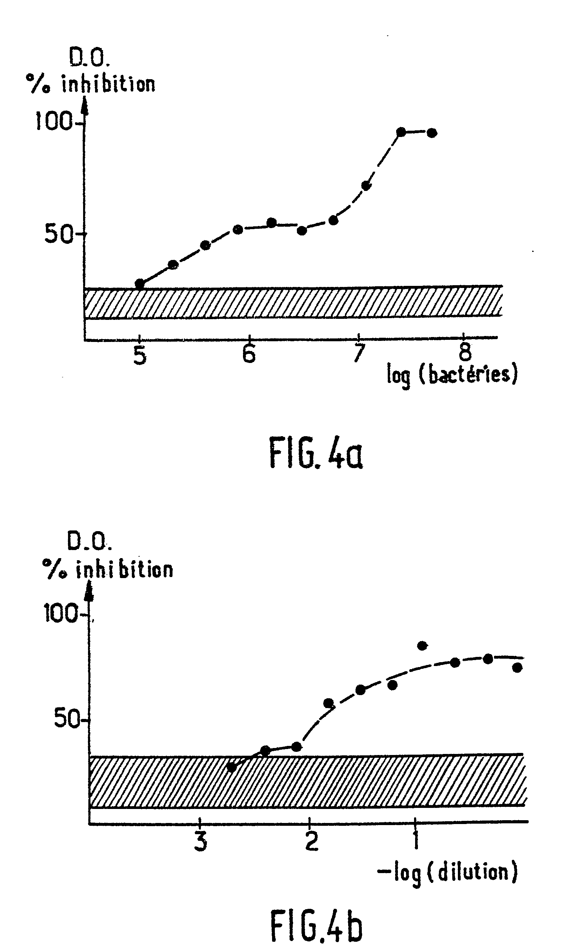

On a utilisé les anticorps monoclonaux dans ces deux types de dosage; les résultats obtenus sont portés sur les figures 4a et 4b.

La figure 4a est relative à un test par compétition avec les bactéries Legionella pneumophila; le fluide ascitique a été utilisé à la dilution de 1/1000 dans des puits contenant 2 millions de bactéries en présence de différentes quantités de bactéries (le logarithme de la quantité de bactéries est en abscisse et la densité optique en % d'inhibition est en ordonnée). Comme on peut le voir sur cette figure, une inhibition de 50% a été obtenue avec 1 à 10 millions de bactéries. Puisque de telles quantités sont facilement présentes dans les expectorations et les lavages bronchotrachéiques des malades, le test ELISA par compétition peut être utilisé pour le diagnostic des légionelloses dans le stade initial de la maladie.

La zone hachurée montre la déviation moyenne et standard des valeurs témoins obtenues par compétition avec Klebsiella pneumoniae.

La figure 4b est relative à un test par compétition avec des dilutions variées d'anti-sérum anti-Legionella de lapin dans des puits contenant 2 millions de bactéries. Sur cette figure on a porté en ordonnée la densité optique en % d'inhibition et en abscisse le logarithme du titre du sérum. La zone hachurée correspond à la déviation moyenne et standard obtenue par compétition avec du sérum de lapin préimmun. Ces résultats montrent que le titrage d'un antisérum par inhibition peut être réalisé.

EXEMPLE 5
Procédé simple et rapide pour la détection des bactéries Legionella dans le milieu environnant.

1. EAU: le prélèvement de l'eau à tester a été réalisé directement sur les sorties d'eau selon le mode opératoire ci-après:

des filtres Millipore® (acrodisc 0,45 µm de la Société GELMAN) ont été fixés sur les arrivées d'eau à l'aide d'une seringue de 5 ml;

a) Après saturation à 37 °C dans du PBS contenant 3% de BSA pendant 1/2 heure, les différents filtres ont été exposés à une solution d'anticorps monoclonal selon l'invention, diluée au 1/50ème final pendant 1 heure à 37 °C.

Trois lavages (3 × 10 ml) ont ensuite été effectués à l'aide de seringues de 5 ml à température ambiante avec une solution de PBS contenant 0,1% de BSA.

b) Les filtres ont ensuite été exposés à une solution d'anticorps (anti-Ig de souris) couplés à la peroxydase (Société Pasteur production) diluée au 1/500ème final à une température de 37 °C pendant 1 heure.

c) Enfin, les filtres ont été lavés (3 × 10 ml) dans un tampon PBS avant d'être exposés au substrat enzymatique ($H_2O_2$ — ABTS commercialisé par Sigma), et la lecture des résultats a été faite au bout de 30 minutes.

Les témoins étaient des fluides (eau stérile Biocedra) dépourvus de toute contamination.

Selon le mode opératoire ci-dessus, on a pu déceler la présence de *Legionella* dans l'eau d'un établissement hospitalier en un temps très court, environ 200 minutes, alors que jusqu'à présent une telle détection nécessitait la mise en culture des bactéries.

2. *AIR*: un prélèvement de l'air à tester a été réalisé directement sur les sorties d'air de différents systèmes de climatisation:

On a utilisé les mêmes filtres Millipore® que ci-dessus et un système simple (entonnoir hermétiquement fixé sur la grille de ventilation) pour recueillir les bactéries au niveau des filtres, c'est-à-dire les bactéries présentes dans l'air pulsé.

On notera que dans les deux cas ci-dessus, les bactéries peuvent être tuées au niveau des filtres en utilisant par exemple une solution de formol qui peut être éliminée facilement après son action bactéricide.

EXEMPLE 6

Dosage de l'antigène dans l'urine des malades:

Les anticorps monoclonaux obtenus selon l'exemple 1 sont utilisés dans un test par compétition de type ELISA. Les anticorps purifiés sont collés sur la surface de puits d'une plaque de plastique à raison de 50 µl d'une solution contenant 10 µg/ml d'anticorps. On met ensuite l'urine dans les puits; après repos d'une nuit à 4 °C, on lave les puits et introduit de l'anticorps couplé à une enzyme pour mettre en œuvre la méthode ELISA. Par la méthode ainsi décrite, on détecte l'équivalent en antigène de 1000 bactéries par échantillon.

**Revendications pour les Etats contractants: BE, CH, DE, FR, GB, IT, LI, LU, NL, SE**

1. Lignées de cellules hybrides murines anti-Legionella pneumophila de sérogroupe I, déposées à la Collection Nationale de Cultures de Micro-organismes de l'Institut PASTEUR, PARIS (France), sous les n° I-219 et I-220, le 14 février 1983.

2. Anticorps monoclonaux produits par les lignées de cellules hybrides murines anti-Legionella pneumophila de sérogroupe I, déposées à la Collection Nationale de Cultures de Micro-organismes de l'Institut PASTEUR, PARIS (France) sous les n° I-219 et I-220 le 14 février 1983.

3. Anticorps monoclonaux produits par la lignée de cellules hybrides murines déposées à la Collection Nationale de Cultures de Micro-organismes de l'Institut PASTEUR, PARIS (France) sous le n° I-220, caractérisés par le fait qu'ils sont isotypes γ-3.

4. Anticorps monoclonaux produits par la lignée de cellules hybrides murines déposées à la Collection Nationale de Cultures de Micro-organismes de l'Institut PASTEUR, PARIS (France) sous le n° I-219, caractérisés par le fait qu'ils sont isotypes γ-2b.

5. Application des anticorps monoclonaux anti-Legionella pneumophila tels que définis à l'une des revendications 2 à 4, au diagnostic rapide des pneumonies causées par les bactéries Legionella pneumophila.

6. Coffret de diagnostic des Legionella pneumophila par méthode immuno-enzymatique ou par immunofluorescence, caractérisé par le fait qu'il contient, à titre de réactif immunologique, un anticorps monoclonal tel que défini à l'une des revendications 2 à 4.

7. Immuno-adsorbants sur lesquels sont fixés des anticorps monoclonaux tels que définis à l'une des revendications 2 à 4.

8. Application des anticorps monoclonaux anti-Legionella pneumophila tels que définis à l'une des revendications 2 à 4, au dépistage épidémiologique.

9. Application des anticorps monoclonaux anti-Legionella pneumophila tels que définis à l'une des revendications 2 à 4, à la préparation de vaccins.

**Revendications pour l'Etat contractant: AT**

1. Lignées de cellules hybrides murines anti-Legionella pneumophila de sérogroupe I, déposées à la Collection Nationale de Cultures de Microorganismes de l'Institut PASTEUR, PARIS (France), sous les n° I-219 et I-220, le 14 février 1983.

2. Procédé de préparation d'anticorps monoclonaux anti-Legionella, caractérisé par le fait que lesdits anticorps sont produits par des cellules hybrides murines anti-Legionella pneumophila de sérogroupe I, des lignées ayant fait l'objet des dépôts n° I-219 et n° I-220, le 14 février 1983, à la Collection Nationale de Cultures de Micro-organismes de l'Institut PASTEUR, PARIS (France).

3. Procédé selon la revendication 2, caractérisé par le fait que les anticorps produits par les cellules de la lignée CNCM I-219 sont isotypes γ-2b.

4. Procédé selon la revendication 2, caractérisé par le fait que les anticorps produits par les cellules de la lignée CNCM I-220 sont isotypes γ-3.

5. Procédé de dosage qualitatif et quantitatif de bactéries Legionella pneumophila dans les liquides biologiques, caractérisé par le fait qu'il met en œuvre les anticorps préparés par le procédé tel que défini à l'une des revendications 2 à 4.

6. Procédé de détection de bactéries Legionella pneumophila dans l'environnement, caractérisé par le fait qu'il met en œuvre des anticorps préparés par le procédé tel que défini à l'une des revendications 2 à 4.

7. Procédé de préparation de vaccins anti-Legionella pneumophila, caractérisé par le fait qu'il met en œuvre des anticorps monoclonaux obtenus par le procédé tel que défini à l'une des revendications 2 à 4.

8. Coffret de diagnostic de Legionella pneumophila par méthode immunoenzymatique ou par immunofluorescence, caractérisé par le fait qu'il contient, à titre de réactif immunologique, un anticorps monoclonal préparé par le procédé tel que défini à l'une des revendications 2 à 4.

9. Immuno-adsorbants sur lesquels sont fixés des anticorps monoclonaux préparés par le procédé tel que défini à l'une des revendications 2 à 4.

**Claims for the contracting States: BE, CH, DE, FR, GB, IT, LI, LU, NL, SE**

1. Mouse anti-Legionella pneumophila serogroup I hybrid cell lines, deposited with the Collection Nationale de Cultures de Micro-organismes [National Collection of Microorganism Cultures] of the Institut PASTEUR, PARIS (France), under nos. I-219 and I-220 on 14th February 1983.

2. Monoclonal antibodies produced by the mouse anti-Legionella pneumophila serogroup I hybrid cell lines deposited with the Collection Nationale de Cultures de Micro-organismes [National Collection of Microorganism Cultures] of the Institut PASTEUR, PARIS (France), under nos. I-219 and I-220 on 14th February 1983.

3. Monoclonal antibodies produced by the mouse hybrid cell line deposited with the Collection Nationale de Cultures de Micro-organismes [National Collection of Microorganism Cultures] of the Institut PASTEUR, PARIS (France), under no. I-220, characterized in that they are γ-3 isotypes.

4. Monoclonal antibodies produced by the mouse hybrid cell line deposited with the Collection Nationale de Cultures de Micro-organismes [National Collection of Microorganism Cultures] of the Institut PASTEUR, PARIS (France), under no. I-219, characterized in that they are γ-2b isotypes.

5. Application of the anti-Legionella pneumophila monoclonal antibodies, as defined in one of Claims 2 to 4, to the rapid diagnosis of pneumonia caused by Legionella pneumophila bacteria.

6. Kit for the diagnosis of Legionella pneumophila by an immunoenzymatic method or by immunofluorescence, characterized in that it contains, by way of an immunological reagent, a monoclonal antibody as defined in one of Claims 2 to 4.

7. Immunoadsorbants to which are bound monoclonal antibodies as defined in one of Claims 2 to 4.

8. Application of the anti-Legionella pneumophila monoclonal antibodies, as defined in one of Claims 2 to 4, to epidemiological screening.

9. Application of the anti-Legionella pneumophila monoclonal antibodies, as defined in one of Claims 2 to 4, to the preparation of vaccines.

**Claims for the contracting State: AT**

1. Mouse anti-Legionella pneumophila serogroup I hybrid cell lines, deposited with the Collection Nationale de Cultures de Micro-organismes [National Collection of Microorganism Cultures] of the Institut PASTEUR, PARIS (France), under nos. I-219 and I-220 on 14th February 1983.

2. Process for preparing anti-Legionella monoclonal antibodies, characterized in that the said antibodies are produced by mouse anti-Legionella pneumophila serogroup I hybrid cells of the lines which form the subject of the depositions no. I-219 and no. I-220, on 14th February 1983, with the Collection Nationale de Cultures de Micro-organismes [National Collection of Microorganism Cultures] of the Institut PASTEUR, PARIS (France).

3. Process according to Claim 2, characterized in that the antibodies produced by the cells of CNCM line I-219 are γ-2b isotypes.

4. Process according to Claim 2, characterized in that the antibodies produced by the cells of CNCM line I-220 are γ-3 isotypes.

5. Process for the qualitative and quantitative assay of Legionella pneumophila bacteria in biological fluids, characterized in that it employs the antibodies prepared by the process as defined in one of Claims 2 to 4.

6. Process for the detection of Legionella pneumophila bacteria in the environment, characterized in that it employs antibodies prepared by the process as defined in one of Claims 2 to 4.

7. Process for preparing anti-Legionella pneumophila vaccines, characterized in that it employs monoclonal antibodies obtained by the process as defined in one of Claims 2 to 4.

8. Kit for the diagnosis of Legionella pneumophila by an immunoenzymatic method or by immunofluorescence, characterized in that it contains, by way of an immunological reagent, a monoclonal antibody prepared by the process as defined in one of Claims 2 to 4.

9. Immunoadsorbants to which are bound monoclonal antibodies prepared by the process as defined in one of Claims 2 to 4.

**Patentansprüche für die Vertragsstaaten: BE, CH, DE, FR, GB, IT, LI, LU, NL, SE**

1. Hybride Mäuse-Zellinien anti-Legionella pneumophila der Serumgruppe I, die in der Collection Nationale de Cultures de Micro-organismes des Institut PASTEUR, Paris, Frankreich, unter der Nummer I-219 und I-220 am 14. Februar 1983 hinterlegt wurden.

2. Monoklonale Antikörper, produziert durch die hybriden Mäusezellen anti-Legionella pneumophila der Serumgruppe I, wobei diese Zellinien unter den Nummern I-219 und I-220 am 14. Februar 1983 in der Collection Nationale de Cultures de Micro-organismes des Institut PASTEUR, Paris, Frankreich, hinterlegt wurden.

3. Monoklonale Antikörper, produziert durch die in der Collection Nationale de Cultures de Micro-organismes des Institut PASTEUR, Paris, Frankreich, unter der Nummer I-220 hinterlegte Linie von hybriden Mäusezellen, dadurch gekennzeichnet, daß sie Isotypen γ-3 sind.

4. Monoklonale Antikörper, produziert durch die in der Collection Nationale de Cultures de Micro-organismes des Institut PASTEUR, Paris, Frankreich, unter der Nummer I-219 hinterlegte Linie von hybriden Mäusezellen, dadurch gekennzeichnet, daß sie Isotypen γ-2b sind.

5. Anwendung der monoklonalen Antikörper anti-Legionella pneumophila, wie sie in einem der Ansprüche 2 bis 4 definiert sind, zur schnellen Diagnostik von Pneumonien, die durch die Bakterien Legionella pneumophila verursacht worden sind.

6. Kit zur Diagnose von Legionella pneumophila mit Hilfe der immunoenzymatischen Methode oder mit Hilfe von Immunofluoreszenz, dadurch gekennzeichnet, daß der Kit als immunologisches Reagenz einen monoklonalen Antikörper enthält, wie er in einem der Ansprüche 2 bis 4 definiert ist.

7. Immunoadsorbentien, auf denen die monoklonalen Antikörper, wie sie in einem der Ansprüche 2 bis 4 definiert sind, fixiert sind.

8. Anwendung der monoklonalen Antikörper anti-Legionella pneumophila, wie sie in einem der Ansprüche 2 bis 4 definiert sind, zum epidemiologischen Nachweis.

9. Anwendung der monoklonalen Antikörper anti-Legionella pneumophila, wie sie in einem der Ansprüche 2 bis 4 definiert sind, zur Herstellung von Vakzinen.

**Patentansprüche für den Vertragsstaat: AT**

1. Hybride Mäuse-Zellinien anti-Legionella pneumophila der Serumgruppe I, die in der Collection Nationale de Cultures de Micro-organismes des Institut PASTEUR, Paris, Frankreich, unter der Nummer I-219 und I-220 am 14. Februar 1983 hinterlegt wurden.

2. Verfahren zur Herstellung von anti-Legionella monoklonalen Antikörpern, dadurch gekennzeichnet, daß diese Antikörper durch die hybriden Mäusezellen anti-Legionella pneumophila der Serumgruppe I produziert werden, wobei diese Zellinien unter den Nummern I-219 und I-220 am 14. Februar 1983 in der Collection Nationale de Cultures des Micro-organismes des Institut PASTEUR, Paris, Frankreich, hinterlegt wurden.

3. Verfahren nach Anspruch 2, dadurch gekennzeichnet, daß die Antikörper, die durch die Zellen der Linie CNCM I-219 produziert werden, Isotypen γ-2b sind.

4. Verfahren nach Anspruch 2, dadurch gekennnzeichnet, daß die Antikörper, die durch die Zellen der Linie CNCM I-220 produziert werden, Isotypen γ-3 sind.

5. Verfahren zur qualitativen und quantitativen Analyse von Legionella pneumophila Bakterien in biologischen Flüssigkeiten, dadurch gekennzeichnet, daß man die Antikörper verwendet, die durch das Verfahren, wie es in einem der Ansprüche 2 bis 4 definiert ist, hergestellt werden.

6. Verfahren zum Auffinden von Legionella pneumophila Bakterien in der Umwelt, dadurch gekennzeichnet, daß man Antikörper verwendet, die durch das Verfahren, wie es in einem der Ansprüche 2 bis 4 definiert ist, hergestellt wurden.

7. Verfahren zur Herstellung von anti-Legionella pneumophila Vakzinen, dadurch gekennzeichnet, daß man monoklonale Antikörper verwendet, die durch das Verfahren, wie es in einem der Ansprüche 2 bis 4 definiert ist, erhalten werden.

8. Kit zur Diagnose von Legionella pneumophila mit Hilfe der immunoenzymatischen Methode oder mit Hilfe von Immunofluoreszenz, dadurch gekennzeichnet, daß der Kit als immunologisches Reagenz einen monoklonalen Antikörper enthält, der durch das Verfahren, wie es in einem der Ansprüche 2 bis 4 definiert ist, hergestellt wird.

9. Immunoadsorbens, auf dem die monoklonalen Antikörper fixiert sind, die durch das Verfahren, wie es in einem der Ansprüche 2 bis 4 definiert ist, hergestellt werden.

FIG.1

FIG.2

FIG.3a

FIG.3b

FIG. 4a

FIG. 4b